# FASCICULE DE BREVET EUROPEEN

(11) **EP 1 418 956 B1**
(45) Date de publication et mention de la délivrance du brevet: **29.06.2005**
(21) Numéro de dépôt: 02772469.9
(22) Date de dépôt: 29.07.2002
(51) Int. Cl.: A61L 31/04, A61L 27/20, A61L 33/08

(54) **ENDOPROTHESE VASCULAIRE RECOUVERTE D'UN DERIVE FONCTIONNALISE DE DEXTRANE**
GEFÄSSPROTHESE BESCHICHTET MIT EINEM FUNKTIONELLEM DEXTRAN-DERIVATE
ENDOVASCULAR PROSTHESIS COATED WITH A FUNCTIONALISED DEXTRAN DERIVATIVE

(30) Priorité: 27.07.2001 FR 0110199; 19.10.2001 FR 0113540
(43) Date de publication de la demande: 19.05.2004
(73) Titulaire: Floréane Médical Implants, 01600 Trévoux (FR)
(72) Inventeur: LEFRANC, Olivier, F-60110 LORMAISON (FR); AVRAMOGLOU, Thierry, F-95170 DEUIL-LA-BARRE (FR); JOZEFONVICZ, Jacqueline, F-60260 LAMORLAYE (FR); DARNIS, Thierry, F-01480 FRANS (FR); THERIN, Michel, F-69004 LYON (FR)
(74) Mandataire: Brédeville, Odile Marie
(86) Numéro de dépôt international: PCT/FR2002/002722
(87) Numéro de publication internationale: WO 2003/011355

(56) Documents cités:
- WO-A-83/03977
- WO-A-93/09790
- GB-A- 2 325 934
- US-A- 4 908 075

## Description

La présente invention a trait au domaine des endoprohèses métalliques et plus particulièrement aux stents utilisés dans le traitement des maladies sténosantes.

L'utilisation des stents s'est considérablement accrue depuis ces cinq dernières années et représente, aujourd'hui, la large majorité des angioplasties coronariennes transluminales percutanées.

Avant l'utilisation des stents, trois quarts ou plus, des patients ayant subi une angioplastie coronarienne transluminale percutanée étaient sujets à la resténose et nécessitaient une réintervention.

L'utilisation des stents a considérablement réduit ce pourcentage. Toutefois, un taux de resténose important (environ 30%) subsiste malgré l'utilisation de tels implants.

Pour rendre ces endoprothèses métalliques plus efficaces et mieux tolérées, leur surface peut être modifiée, par le biais de traitements chimiques ou radioactifs, et/ou recouverte de substances d'origines biologiques (héparine, phosphorylcholine, ADN), de polymères (silicones, polyuréthannes, polytétrafluoroéthylene expansé) ou des céramiques (TiO₂, C...). D'autres endoprothèses sont constituées de carbone et même de matériaux biodégradables.

Les différentes modifications de surface peuvent dans certains cas améliorer le comportement *in vivo* des stents mais ne suffisent pas à ce jour à prévenir des complications telles que la resténose. Par ailleurs, un certain nombre d'entre elles peuvent conduire à une dégradation substantielle des propriétés mécaniques du stent sur lequel elles sont appliquées.

On connaît de EP-B-716836 des prothèses métalliques, dont des endoprothèses pour angioplastie qui sont enrobées d'un film de polymère permettant la délivrance in-situ, par biodégradation du polymère, de principes actifs pharmaceutiques.

De FR-A-2785812 on connaît l'immobilisation sur une surface d'endoprothèse métallique, par dépôt par électropolymérisation, de polymères acryliques, qui permettrait ensuite la fixation et la diffusion de principes actifs. Aucun exemple de fixation de principe actif et de diffusion en conservant les propriétés biologiques du principe actif n'est mentionné dans ce brevet.

Dans ces deux brevets, outre le fait que les principes actifs pharmaceutiques seraient nécessairement libérés pour avoir une activité biologique, les polymères qui permettent leur fixation sont déposés en couches sur des surfaces métalliques, ces couches pouvant présenter, comme exposé précédemment, un risque de délamination. De plus du fait de la biodégradabilité du polymère et de la diffusion du principe actif, les effets biologiques sont nécessairement limités dans le temps.

On citera également de WO-A-9746590, l'immobilisation de substances bioactives permettant la modification des propriétés de polymères sur la surface desquels cette immobilisation est effectuée, par exemple pour leur conférer des propriétés antithrombotiques et en outre rendre leur surface hydrophile ; ces polymères sont utilisés pour la fabrication de prothèses vasculaires par exemple en PTFE expansé.

On connaît de WO 99/29734 ou de l'article de D. Logeart-Avramoglou et J. Jozefonvicz paru dans J. Biomed. Mater. Res. (Appl. Biomater) 48, 578-590, 1999 des dérivés de dextrane fonctionnalisés qui répondent à la formule générale DMCₐB_{b}Su_{c}S_{d} dans laquelle :
D représente une chaîne polysaccharidique, constituée par des enchaînements d'unités α-D-glucopyranosiques reliées entre elles par des liaisons α(1-6),
MC représente des groupes méthylcarboxylates,
B représente des groupes carboxyméthylbenzylamides,
Su représente des groupes sulfates,
S représente des groupes sulfonates, et
a, b, c et d représentent le degré de substitution (ds), exprimé par rapport au nombre de fonctions hydroxyles libres dans une unité glucosidique du dextrane, respectivement en groupements MC, B, Su et S, a étant égal à 0 ou ≥ à 0,2, b étant égal à 0 ou ≥ à 0,1, c étant égal à 0 ou ≥ à 0,1 et d étant égal à 0 ou ≤ à 0,15, à condition que lorsque d=0, a et/ou b soient ≠ 0.

On connaît également de EP-A-146455, des dérivés de dextrane qui comportent de manière statistique :
- au moins 35% environ de motifs B constitués de motifs osides A substitués par des radicaux possédant une fonction carboxyle répondant a la structure -O-(CH₂)ₙ-R-COO⁻ dans laquelle R représente une simple liaison ou un groupe -CO-NH-(CH₂)_{n'}⁻, n étant un nombre compris entre 1 et 10 et n' étant compris entre 1 et 7.
- au moins 3% environ de motifs D, c'est à dire de motifs osides A substitués par une chaîne comportant un groupe de structure : 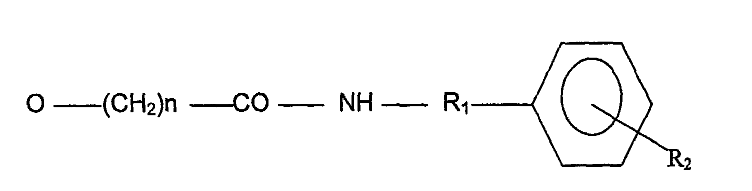
dans laquelle n est défini ci-dessus, R2 représente un anion d'un sel minéral ou organique physiologiquement tolérable, et R₁ représente une simple liaison, un groupe -CH₂- ou un groupe : 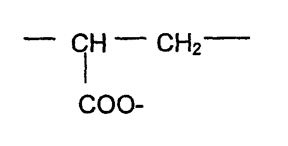
- éventuellement, des motifs osides A non substitués et/ou des motifs C constitués de motifs A substitués par des radicaux de structure suivante, dans laquelle R₁ et n sont tels que définis ci-dessus :

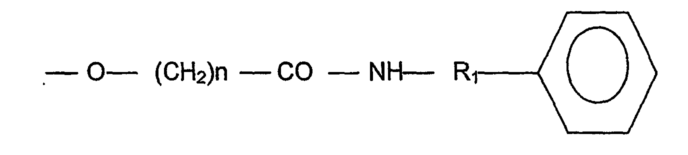

On connaît également de EP-A-0428182, des dérivés de dextrane d'un poids moléculaire supérieur à environ 5000 g/mol, dotés d'une forte activité anti-complémentaire et d'une activité anti-coagulante faible, qui comprennent des motifs A et C et au moins 35% de motifs B, ces motifs étant tels que définis ci-dessus dans le brevet EP-A-0146455.

La présence de dérivés fonctionnalisés de dextrane à la surface de l'endoprothèse permet de développer sur la surface de cette dernière des interactions spécifiques avec le milieu biologique dans lequel elle est implantée ; on observe notamment une inhibition de la prolifération des cellules musculaires lisses humaines et une prolifération des cellules endothéliales au contact de l'endoprothèse, processus qui favorise l'intégration de l'endoprothèse dans le milieu biologique.

En outre, en fonction de leurs degrés de substitution en différents groupements fonctionnalisés, les dérivés fonctionnalisés de dextrane et les polysaccharides fonctionnalisés décrits ci-dessus peuvent présenter des activités anti-complémentaires et de substitut de plasma sanguin, une activité modulatrice de la prolifération des cellules musculaires lisses et endothéliales ou encore des propriétés anticoagulantes ou une action vis-à-vis des plaquettes.

Le but de la présente invention est donc de conférer à un substrat métallique, pouvant être utilisé comme endoprothèse, par exemple stent, des propriétés biologiques d'intérêt, compte tenu de l'application ou indication de ladite prothèse, et ce de manière complètement intégrée au substrat métallique et permanente, c'est à dire sans modifier les propriétés mécaniques intrinsèques dudit substrat d'une part, et en fixant définitivement sur ce substrat les agents ou composés actifs retenus pour exhiber les propriétés biologiques précitées, d'autre part.

Un premier objet de la présente invention consiste donc en un procédé de revêtement et liaison de la surface d'un substrat métallique avec une couche d'un composé polysaccharidique, caractérisé en ce que à partir du substrat métallique :
(a) on dispose d'un agent de modification chimique de la surface du substrat métallique, par exemple sous forme liquide ;
(b) on dispose d'un agent de recouvrement intermédiaire, comprenant un composé silanique, en solution par exemple, comportant deux restes réactifs, l'un avec le substrat métallique, et l'autre, directement ou indirectement avec le composé polysaccharidique ;
(c) on dispose d'un agent de revêtement, comprenant, en solution par exemple, le composé polysaccharidique ;
et on effectue les opérations suivantes :
(1) on met en contact la surface du substrat métallique, avec l'agent de modification chimique, pour obtenir une surface modifiée chimiquement ;
(2) on met en contact la surface modifiée chimiquement avec l'agent de recouvrement intermédiaire, pour obtenir une surface revêtue avec une couche intermédiaire comprenant le composé silanique, lié par covalence au substrat métallique ;
(3) on met en contact la couche intermédiaire avec l'agent de revêtement, pour revêtir ladite couche intermédiaire avec un revêtement comprenant le composé polysaccharidique lié par covalence, directement ou indirectement, au composé silanique.

Selon l'invention l'étape (3) de mise en contact de la couche intermédiaire avec l'agent de revêtement peut être répétée pour améliorer l'épaisseur de la couche de revêtement comprenant le composé polysaccharidique lié par covalence, directement ou indirectement, au composé silanique.

Selon l'invention le substrat métallique obtenu à l'étape (2) du procédé peut être isolé, le procédé selon l'invention consiste ainsi en un procédé de revêtement et liaison de la surface d'un substrat métallique avec une couche d'un agent de recouvrement intermédiaire comprenant un composé silanique, caractérisé en ce que à partir du substrat métallique :
(a) on dispose d'un agent de modification chimique de la surface du substrat métallique, par exemple sous forme liquide ;
(b) on dispose d'un agent de recouvrement intermédiaire, comprenant un composé silanique, comportant au moins un reste réactif avec le substrat métallique,
et on effectue les opérations suivantes :
(1) on met en contact la surface du substrat métallique, avec l'agent de modification chimique, pour obtenir une surface modifiée chimiquement ;
(2) on met en contact la surface modifiée chimiquement avec l'agent de recouvrement intermédiaire, pour obtenir une surface revêtue avec une couche comprenant le composé silanique, lié par covalence au substrat métallique.

Un autre objet de la présente invention est un objet métallique à usage médical ou chirurgical, du type prothèse, par exemple endoprothèse vasculaire (dite "stent") pour angioplastie coronarienne transluminale percutanée, comprenant un substrat métallique dont la surface est revêtue au moins en partie avec un composé polysaccharidique, caractérisé en ce que le composé polysaccharidique est lié par covalence au substrat métallique, par l'intermédiaire de bras de greffage, comprenant chacun au moins un motif silanique, lié d'un côté au substrat métallique par une liaison métal -O-, et de l'autre côté, directement ou indirectement, par liaison covalente -NH-, avec le composé polysaccharidique.

Un autre objet de l'invention est un objet métallique à usage médical ou chirurgical, du type prothèse, par exemple endoprothèse vasculaire (dite "stent") pour angioplastie coronarienne transluminale percutanée, comprenant un substrat métallique dont la surface est revêtue au moins en partie un agent de recouvrement comportant un composé silanique, caractérisé en ce que le composé silanique, est lié au substrat métallique par une liaison métal-O-.

Selon l'invention, les composés polysaccharidiques se prêtent bien à un greffage sur un substrat métallique, si l'on utilise un composé silanique au moins bifonctionnalisé, et les propriétés biologiques intrinsèques des composés polysaccharidiques ne sont pas altérées par le greffage.

Ainsi l'invention permet d'obtenir un objet métallique à usage médical ou chirurgical, du type prothèse, par exemple endoprothèse vasculaire (dite "stent") pour angioplastie coronarienne transluminale percutanée, qui présente l'avantage, de conserver toutes ses propriétés mécaniques, qui déclenche une réponse biologique favorable ou, au minimum, ne déclenche pas de réponse biologique défavorable, chez le sujet receveur et donc limite la resténose.

Selon l'invention le procédé permet la conservation des propriétés biologiques du composé polysaccharidique mis en oeuvre par ledit procédé et le composé polysaccharidique conserve ses propriétés biologiques intrinsèques après dépôt.

On entend par propriétés biologiques intrinsèques les activités biologiques précitées et notamment des activités anti-complémentaires et de substitut de plasma sanguin, une activité modulatrice de la prolifération des cellules musculaires lisses et endothéliales ou encore dés propriétés anticoagulantes ou une action vis-à-vis des plaquettes.

Les objets ou les endoprothèses soumis au procédé selon l'invention présentent également comme caractéristique qu'ils n'entraînent pas la diffusion dans l'organisme de produits toxiques.

La non toxicité de la diffusion de produits toxiques dans l'organisme est vérifiée par le test préconisé dans la norme internationale ISO 10 993-5, concernant l'évaluation biologique des dispositifs médicaux.

Un substrat métallique selon l'invention est un support, dont la surface est destinée à recevoir le revêtement selon l'invention, réalisé en métal ou dont la surface est revêtue d'un métal ou d'un alliage comme les aciers inoxydables, les alliages à base de chrome et de cobalt ou même les superalliages.

Par métal selon l'invention, on entend tout matériau constitué d'un corps simple bon conducteur de la chaleur et de l'électricité, ayant un grand pouvoir réflecteur à l'état poli, et donnant des oxydes qui réagissent avec l'eau pour donner des bases comme le fer, le cobalt, le chrome.

Par alliage, on entend tout produit métallique homogène obtenu par l'association de plusieurs métaux avec une nette prédominance de l'un d'entre eux, en vue de conférer à ce dernier des caractéristiques déterminées.

Par composé silanique on entend des composés organiques choisis parmi les dérivés des silanes ayant pour formule générale SiₙH₂ₙ₊₂, dont l'un ou plusieurs atomes d'hydrogène a été substitué par des fonctions organiques classiques comme les groupes alkyles, des groupes alcoxy, amines ou autre fonctions organiques.

Par dérivé d'un silane comprenant une ou plusieurs fonctions réactives, on entend un composé comportant, un ou plusieurs groupes amines ou dérivés d'amines susceptibles d'entrer en réaction avec les groupes hydroxyles des composés osidiques, et comportant un ou plusieurs groupes hydroxyles ou alcoxy capables de réagir, soit directement, soit après hydrolyse avec le métal constitutif du substrat métallique, par exemple les composés choisis dans le groupe constitué par les aminopropylsilanes et les aminobutylsilanes.

Par composé polysaccharidique selon l'invention on entend tout polymère, naturel ou synthétique, comprenant une chaîne polymère constituée d'une multiplicité de motifs osidiques, à savoir tout polysaccharide, non modifié et/ou non fonctionnalisé, ou tout dérivé polysaccharidique fonctionnalisé.

Par composé polysaccharidique fonctionnalisé on entend des dérivés polysaccharidiques comportant des motifs osidiques comportant des fonctions hydroxyles substituées par des groupes comme par exemple des groupes méthylcarboxylates, des groupes carboxyméthylbenzylamide, des groupes sulfates, des groupes sulfonates, ou dont les fonctions hydroxyles ont été substituées par des chaines comportant des fonctions carboxyles, des fonctions amides, des groupes benzyles seuls ou en combinaison.

Plus particulièrement les composés polysaccharidiques seront choisis parmi les composés de formule générale DMCₐB_{b}Su_{c}S_{d} dans laquelle :
D représente une chaîne polysaccharidique, constituée par des enchaînements d'unités α-D-glucopyranosiques reliées entre elles par des liaisons α(1-6),
MC représente des groupes méthylcarboxylates,
B représente des groupes carboxyméthylbenzaylamides,
Su représente des groupes sulfates,
S représente des groupes sulfonates, et
a, b, c et d représentent le degré de substitution (ds), exprimé par rapport au nombre de fonctions hydroxyles libres dans une unité glucosidique du dextrane, respectivement en groupements MC, B, Su et S, a étant égal à 0 ou ≥ à 0,2, b étant égal à 0 ou ≥ à 0,1, c étant égal à 0 ou ≥ à 0,1 et d étant égal à 0 ou ≤ à 0,15, à condition que lorsque d=0, a et/ou b soient ≠ 0.
Parmi ces dérivés fonctionnalisés du dextrane, on peut plus particulièrement utiliser ceux qui sont selectionnés dans le groupe constitué par :
- Les dextranes fonctionnalisés dans lesquels a ≥ 0,7, 0,15 ≤ b ≤ 0,3, 0 ≤ c ≤ 0,15 et d = 0 ou ≤ 0,1 et dont la masse molaire moyenne en poids est comprise entre 5 000 et 200 000 g/mol,
- Les dextranes fonctionnalisés dans lesquels 0,4 ≤ a ≤ 0,8, 0,3 ≤ b ≤ 0,8, 0,1 ≤ c ≤ 0,9 et d = 0 ou ≤ 0,1 et dont la masse molaire moyenne en poids est comprise entre 5 000 et 200 000 g/mol,
- Les dextranes fonctionnalisés dans lesquels a ≥ 0,5, 0,3 ≤ b ≤ 0,5, c = 0 ou ≤ 0,1 et d = 0 ou ≤ 0,1 et dont la masse molaire moyenne en poids est comprise entre 5 000 et 200 000 g/mol,

Dans un autre mode de réalisation les composés polysaccharidiques pourront être choisis parmi les dérivés de dextranes qui comportent de manière statistique :
- au moins 35% environ de motifs B constitués de motifs osides A substitués par des radicaux possédant une fonction carboxyle répondant à la structure -O-(CH₂)ₙ-R-COO⁻ dans laquelle R représente une simple liaison ou un groupe -CO-NH-(CH₂)_{n'}⁻, n étant un nombre compris entre 1 et 10 et n' étant compris entre 1 et 7.
- au moins 3% environ de motifs D, c'est à dire de motifs osides A substitués par une chaîne comportant un groupe de structure : 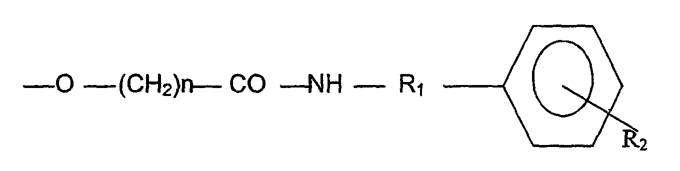
dans laquelle n'est défini ci-dessus, R2 représente un anion d'un sel minéral ou organique physiologiquement tolérable, et R₁ représente une simple liaison, un groupe -CH₂- ou un groupe : 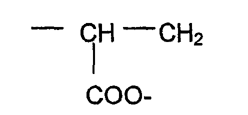
- éventuellement, des motifs osides A non substitués et/ou des motifs C constitués de motifs A substitués par des radicaux de structure suivante, dans laquelle R₁ et n sont tels que définis ci-dessus :

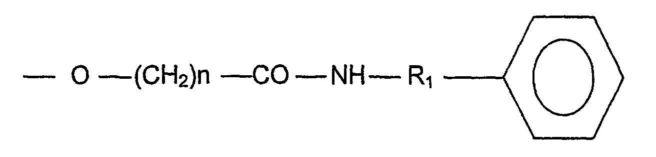

Dans un autre mode de réalisation les composés polysaccharidiques pourront être choisis parmi les dérivés de dextranes, possédant un poids moléculaire supérieur à environ 5000 g/mol, constitués de manière statistique de motifs A, B et C, les motifs A étant des motifs osides de dextranes, les motifs B et C étant tels que définis ci-dessus, qui comprennent des motifs A et C et au moins 35% de motifs B.

Dans un autre mode de réalisation la chaîne polyosidique du composé polysaccharidique est celle d'un polysaccharide choisi dans le groupe constitué par l'amidon, le glycogène, les celluloses, les dextranes, les poly-β-1,3-glucanes, les poly-β-1,6-glucanes, les pullulanes, la chitine, le chitosane, les arabanes, les xylanes, les fucanes, et les pectines, lorsque la chaîne polyosidique du composé polysaccharidique est celle d'un dextrane, elle comprend une multiplicité de motifs α-D-glucopyranosiques reliés entre eux par des liaisons α(1-6).

Dans un autre mode de réalisation le composé polysaccharidique est un polysaccharide naturel ou synthétique, non modifié, en particulier non fonctionnalisé.

Dans le procédé selon l'invention la surface modifiée chimiquement est nettoyée avant mise en contact avec l'agent de recouvrement intermédiaire, par exemple avec une solution d'acétone, ou d'alcool, et/ou d'agents tensioactifs.

Dans le procédé selon l'invention le substrat métallique dont la surface est revêtue avec l'agent de recouvrement intermédiaire est recuite, avant mise en contact avec l'agent de revêtement, ce recuit est effectué à une température comprise entre 80 et 140°C environ, et/ou pendant une durée comprise entre 1 et 30 minutes environ.

Ce recuit permet le retournement de la molécule de dérivé de silane, pour qu'elle présente une fonction amine libre susceptible de réagir avec les composés comportant les groupes hydroxyles.

Par exemple ce retournement peut être illustré pour la molécule de 3-aminopropyltriéthoxysilane (γ-APS) comme suit :

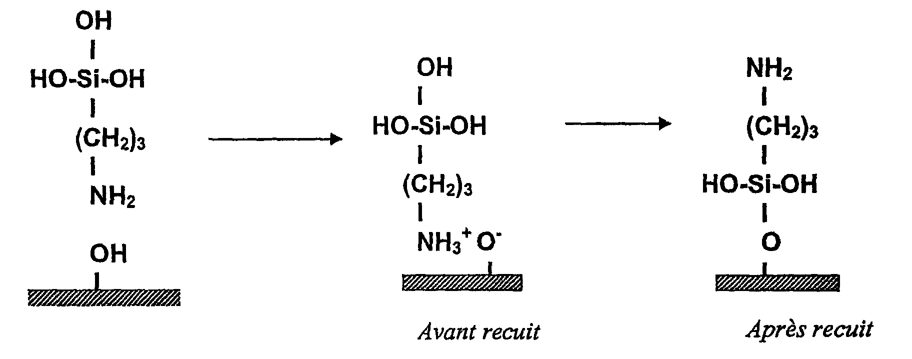

Dans le procédé selon l'invention les surfaces revêtues peuvent être lavées, notamment la surface revêtue de la couche intermédiaire est lavée, avant mise en contact avec l'agent de revêtement, et la surface revêtue avec le composé polysaccharidique est lavée.

Dans le procédé selon l'invention, l'opération (1) et/ou (2) est mise en oeuvre en phase liquide ou vapeur et lorsque l'opération (2) est effectuée en phase liquide, elle est réalisée à un pH compris entre 2 et 9, et/ou à une température comprise entre 25 et 120°C, et/ou pendant une durée comprise entre 1 et 120 minutes.

Lorsque l'opération (2) est effectuée en phase vapeur elle est effectué à une température supérieur à 120 °C et à une pression supérieure à 4 mbar. Lorsque l'opération (2) est ainsi effectuée en phase vapeur la molécule de dérivé de silane présente un fonction amine libre susceptible de réagir avec les composés comportant les groupes hydroxyles pour former une liaison covalente, l'étape de recuit est donc immédiate et consécutive au greffage et ne nécessitera pas une étape supplémentaire de procédé.

L'agent de modification chimique de la surface du substrat métallique, sous forme liquide, comprend au moins un acide minéral fort, par exemple acide sulfurique, ou chlorhydrique, ou nitrique dans la proportion comprise entre 5 et 100 % (VN), et au moins un oxyde de chrome dans la proportion comprise entre 1 et 40 % (m/V) ; de préférence le pH de cet agent de modification chimique est compris entre 1 et 6.

L'oxyde de chrome a une masse molaire moyenne comprise entre 50 et 500 g/mol, et est, par exemple, choisi dans le groupe constitué par les bichromates de potassium et les oxydes de chrome IV.

L'agent de recouvrement intermédiaire, à l'état liquide, comprend au plus 50%, par exemple entre 1 et 30 % (VN) du composé silanique.

Le composé silanique a une masse molaire moyenne comprise entre 50 et 500 g/mol, il est choisi dans le groupe constitué par les aminopropylsilanes, et les aminobutylsilanes.

L'agent du revêtement, à l'état liquide, comprend entre 1 et 20 % (m/V) du composé polysaccharidique, en solution, il comprend un polysaccharide non modifié ou non fonctionnalisé, par exemple un dextrane, et/ou un dérivé polysaccharidique fonctionnalisé, susceptible d'être obtenu à partir d'un polysaccharide, par exemple un dextrane.

Lorsque le polysaccharide est un dextrane, sa masse molaire moyenne est comprise entre 20.000 et 1.000.000 g/mol, par exemple sa masse molaire est égale à 40.000 ou 70.000, ou 460.000 g/mol.

L'agent de revêtement comprend au moins un agent de couplage, par exemple choisi dans le groupe constitué par le bio-sulfo (succinimide-subérate), en abrégé BS3, le diméthyladipimidate, en abrégé DMA, l'époxirane, le bis-époxirane, les succinimides, l'épichlorydrine, les carbodiimides, comme par exemple le 1-éthyl-3-3-(diméthylaminopropyl)-carbodiimide, en abrégé EDAC OU EDC, ou le N-hydroxysuccinimide, en abrégé NHS. De façon préférentielle l'agent de couplage est présent, dans l'agent de revêtement, à proportion de 20 à 50 moles pour 100 moles du motif osidique de la chaîne polysaccharidique.

L'agent de revêtement peut comprendre un polysaccharide supplémentaire, naturel ou synthétique, substitué par des fonctions carboxylates et/ou sulfates, ledit polysaccharide supplémentaire étant différent dudit dérivé polysaccharidique fonctionnalisé.

La présente invention a également pour objet le substrat métallique revêtu, susceptible d'être obtenu par le procédé précédemment décrit et une endoprothèse vasculaire, du type stent, comprenant ce substrat métallique revêtu, dont le matériau constitutif est un alliage, par exemple un acier inoxydable, ou un superalliage, par exemple le phynox.

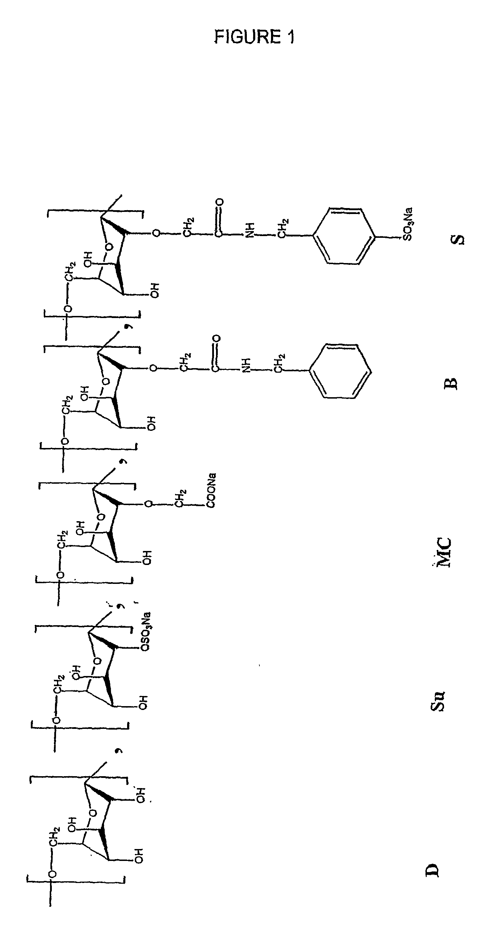

Différents objets de l'invention sont illustrés dans les exemples de mise en oeuvre du procédé et dans les figures 1 à 2 ci-dessous décrites :
- La figure 1 représente schématiquement la structure d'un dérivé fonctionnalisé de dextrane substitué par les différents groupements chimiques MC, B, Su et S fixés su les unités glucosidiques D ; à titre d'exemple, la position du substituant sur les différents carbones des unités glucosidiques est présentée sur le carbone 2 ;
- La figure 2 représente les courbes d'inhibition de la prolifération de cellules musculaires lisses issues d'aorte de rat, au bout de 5 jours d'incubation, en fonction des différents dérives fonctionnalisés de dextrane.

### EXEMPLE 1 : Préparation de modèles pour endoprothèses vasculaires en acier inoxydable selon la présente invention, nettoyage, oxydation et amination de la surface.

### 1) Support, solutions d'attaque et dérivés silaniques utilisés

Le support utilisé consiste en des pions d'acier inoxydable 316L polis de diamètre 8 mm et d'épaisseur 3 mm fournis par la société Sofradim.

Ces pions servent de modèle pour les endoprothèses vasculaires elles mêmes en acier inoxydable 316L.

L'acier inox 316L est un acier austénitique type 18/12 (teneur en Chrome/Nickel), à structure CFC répondant aux normes européennes NF S 90 401, NF S 90 402, NF S 90 403 et NF S 94 051.

Les solutions de nettoyage sont des solutions d'acétone et d'éthanol chaud. La solution oxydante est un mélange sulfochromique.

Le composé silanique, est le γ-APS (3-aminopropyl)triéthoxysilane. Ce composé se fixe sur les fonctions hydroxyles de la surface et permet d'obtenir les fonctions amines apparentes.

### 2) Protocole

Les pions d'acier inox 316L sont soumis à la sonication dans l'acétone 10 mn puis placés 10 mn dans une solution d'éthanol à 70°C.

Cette étape permet l'élimination des éléments tensioactifs pour le nettoyage.

Une solution de γ-APS à 10% (V/v) dans l'éthanol 95° est préparée et laissée 1 heure sous agitation afin d'hydrolyser les fonctions éthoxy et de permettre un greffage sur des fonctions hydroxyles.

La solution d'oxydation est une solution sulfochromique à 2,7% (MN) en bichromate de potassium dans l'acide sulfurique à 80%.

Dans le même temps, les pions d'acier nettoyés sont placés dans la solution d'oxydation et laissés 1 heure sous faible agitation.

Les pions oxydés sont ensuite rincés dans l'eau bidistillée, 5 minutes sous sonication.

Les pions ainsi récupérés sont alors placés dans la solution de γ-APS et laissés 1 heure sous faible agitation.

On effectue ensuite un recuit des pions soumis au γ-APS pour permettre le retournement de la molécule de γ-APS selon la réaction illustrée précédemment.

La phase aqueuse est soigneusement retirée à l'aide d'une pipette sans entrer en contact avec les pions d'acier puis le récipient contenant les pions est placé 10 minutes à 120°C.

Cette étape de recuit permet la fixation du γ-APS à la surface des pions.

Les pions sont finalement rincés à l'eau bidistillée et conservés à 50°C.

### EXEMPLE 2 : Préparation de modèles pour endoprothèses vasculaires en phynox, nettoyage, oxydation et amination de la surface

### 1) Support, solutions d'attaque et composés silaniques utilisés

Le support utilisé consiste en des pions de Phynox poli-miroir de diamètre 10 mm et d'épaisseur 4 mm fournis par la société Sofradim.

Ces pions servent de modèle pour les endoprothèses vasculaires elles-mêmes en phynox.

Le phynox est un superalliage à base de cobalt dont la résistance à l'oxydation est bien meilleure que celle des aciers inoxydables.

Le phynox répond aux exigences des normes ASTM F-91 et ISO 5832/7 et NF S 94-057 concernant les implants chirurgicaux.

Les solutions de nettoyage sont des solutions d'acétone et d'éthanol chaud.

La solution oxydante est un mélange sulfochromique.

Le composé silanique, est le γ-APS (3aminopropyltriéthoxysilane). Ce composé se fixe sur les fonctions hydroxyles de la surface et permet d'obtenir les fonctions amines apparentes.

### 2) Protocole

Les pions de phynox sont soumis à la sonication dans l'acétone 10 mn puis placés 10 mn dans une solution d'éthanol a 70°C.

Cette étape permet l'élimination des éléments tensioactifs utilisés pour le nettoyage.

Une solution de γ-APS à 10% (V/V) dans l'éthanol 95° est préparée et laissée 1 heure sous agitation afin d'hydrolyser les fonctions éthoxy et de permettre un greffage sur des fonctions hydroxyles.

La solution d'oxydation est une solution sulfochromique à 2,7% (mN) en bichromate de potassium dans l'acide sulfurique 80%.

Dans le même temps, les pions d'acier nettoyés sont placés dans la solution d'oxydation et laissés 1 heure sous faible agitation.

Les pions oxydés sont ensuite rincés dans l'eau bidistillée, 5 minutes sous sonication.

Les pions ainsi récupérés sont alors placés dans la solution de γ-APS et laissés 1 heure sous faible agitation.

On effectue ensuite un recuit des pions soumis au γ-APS pour permettre le retournement de la molécule de γ-APS selon la réaction illustrée précédemment.

La phase aqueuse est soigneusement retirée à l'aide d'une pipette sans entrer en contact avec les pions d'acier puis le récipient contenant les pions est placé 10 minutes à 120°C.

Cette étape de recuit permet la fixation du γ-APS à la surface des pions.

Les pions sont alors rincés à l'eau bidistillée et conservés à 50°C.

### EXEMPLE 3 : Préparation d'objets métalliques pour endoprothèses avec amination de la surface en phase vapeur.

Le système est composé de deux enceintes séparées par un robinet. L'enceinte destinée à accueillir le γ-APS est reliée à une ampoule à brome.

Les objets métalliques préalablement soumis à une oxydation telle que décrite dans les protocoles des exemples 1 ou 2, sont placés dans une enceinte sous faible pression (8 mbar) et portés à 120 °C par un ruban chauffant.

Le γ-APS en solution à 10 % V/V dans de l'éthanol pur est introduit dans l'ampoule à brome, puis injecté dans une des deux enceintes prélablement portée à 140 °C sous une pression de 8 mbar. Le γ-APS est immédiatement vaporisé.

Les enceintes sont ensuite mise en contact, sous une pression de 8 mbar et le γ-APS gazeux est alors mis en contact avec l'objet métallique.

Le greffage est immédiat et ce greffage est effectué de manière covalente car le substrat métallique étant porté à une température de 120 °C l'étape de recuit est immédiate.

Après réaction la pression est ramenée à la pression atmosphérique et l'objet métallique est rincé à l'eau.

Des analyses XPS effectuées sur les différents objets métalliques aminés en phase vapeur ont montré une couche aminée de faible épaisseur sur toute la surface desdits objets.

### EXEMPLE 4 : Analyse de la surface des pions aminés.

### 1) Description des appareils

Afin de vérifier la présence d'un revêtement aminé à la surface des pions, ceux-ci sont observés et leurs surfaces sont caracterisées chimiquement.

Les méthodes d'analyse employées sont L'XPS (X-Photoelectron Spectroscopy) et l'AFM (Microscope à Force Atomique).

### 2) Protocole

L'appareil XPS employé est un Escalab 210, utilisant comme source monochromatique la raie Kα de l'aluminium d'énergie 1486 eV sous ultravide. Cet appareil analyse une surface de 3 mm² (3 mm.lmm) sur une profondeur de 10 nm.

L'AFM est utilisé en mode semi-contact sur la surface des pions d'acier.

### 3) Résultats

L'analyse XPS des pions d'acier inox 316L a montré une couche d'amination homogène sur toute la surface du pion et d'épaisseur supérieure à 10 nm. Cette épaisseur a été estimée à 30 nm. Les fonctions amines libres sont apparentes et permettent ainsi un greffage ultérieur de dextrane et/ou de dérivés fonctionnalisés de dextrane. L'analyse à l'AFM a montré des amas de dérivés silaniques sur les pions si la silanisation n'a pas lieu dans l'éthanol 95° mais dans l'eau. Une analyse identique a montré une couche d'amination homogène sur la surface du pion analysé.

### EXEMPLE 5 : Test de cytotoxicité du revêtement par essai de «diffusion des extraits».

### 1) Test, cellules, témoins et échantillons utilisés

Le test employé est un test de mesure indirecte basé sur la toxicité des produits diffusés ou "relargués" par les pions modifiés. Ce test suit la norme internationale ISO 10 993-5, concernant l'évaluation biologique des dispositifs médicaux.

Les supports utilisés sont des plaques de culture cellulaires 24 puits commercialisées par Corning Costar.

Les cellules employées sont des cellules endothéliales humaines immortalisées de la lignée EAhy 926 en phase de croissance exponentielle ensemencées a 5 000 cellules/puits.

Le véhicule d'extraction, ainsi que le témoin négatif, est un milieu de culture a 10% (V/V) en sérum de veau foetal (SVF).

Le témoin positif est une solution de DMSO a 1 % (V/V) dans du milieu de culture.

Les échantillons utilisés sont des pions d'acier inox 316L bruts, oxydés et aminés par les méthodes décrites ci-dessus.

### 2) Protocole

Les pions d'acier sont stérilisés à l'éthanol 70° durant 30 minutes puis lavés rapidement au tampon PBS stérile. Ils sont ensuite placés chacun au fond d'un puits de plaque de culture cellulaire et recouverts de 1,5 ml de milieu de culture a 10% de SVF. Les plaques sont ensuite placées pendant 72 heures dans un incubateur air/CO2.

Après 72 heures d'incubation, les extraits sont récupérés et mis au contact des cellules EAhy 926 en début de phase de croissance exponentielle.

L'estimation de la densité cellulaire est réalisée au bout de 8 jours d'incubation à l'aide d'un compteur de cellules (coulter counter).

### 3) Résultats

Le tableau 1 donne les différentes valeurs de nombre de cellules par puits au bout de huit jours d'incubation.

| **Acier brut** | Acier oxydé | Acier aminé | Témoin positif | Témoin négatif |
|---|---|---|---|---|
| 90 000 | 95 000 | 95 000 | 45 000 | 100 000 |

Aucun effet cytotoxique n'est observé. Quel que soit le traitement subit, les pions en acier 316L ne diffusent ou ne "relarguent" aucune substance toxique ou inhibitrice de la prolifération cellulaire.

### EXEMPLE 6 : Greffage d'un dérivé fonctionnalisé du dextrane sur des pions d'acier aminés.

### 1) Support métallique, dextrane et agents de couplages utilisés

Les supports métalliques utilisés sont identiques à ceux décrits dans l'exemple 4.

Les agents de couplages utilisés sont l'EDAC et le NHS, commercialisés par Sigma-Aldrich.

Le dérivé fonctionnalisé de dextrane utilisé, de masse molaire moyenne en poids 70 000 g/mol, est le DMC_{0,8}B_{0,22}Su_{0,11} ; il répond à la formule générale DMCₐB_{b}Su_{c}S_{d} telle que celle précédemment décrite.

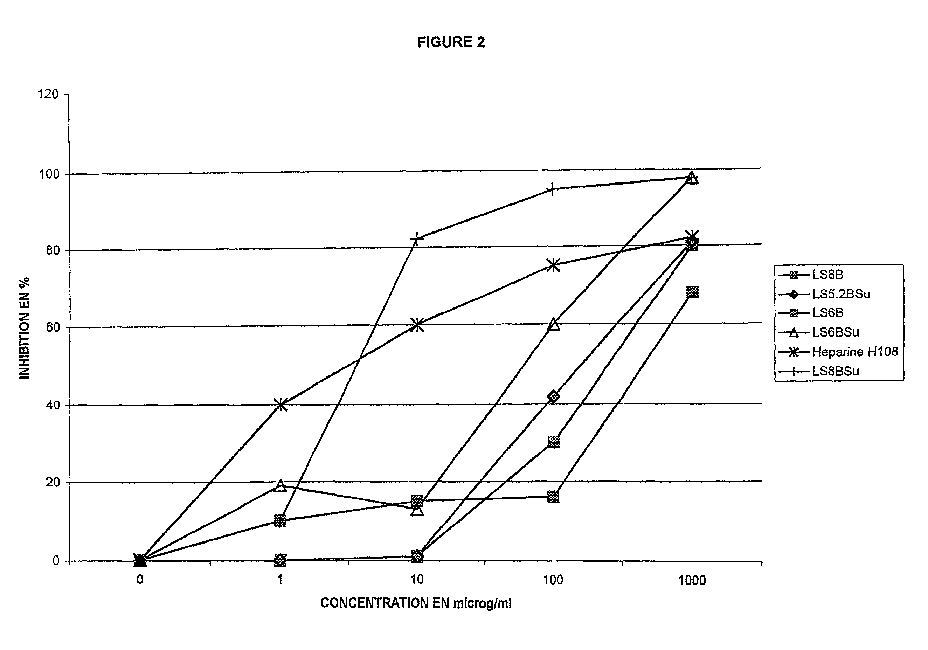

Son protocole de préparation est tel que décrit dans le brevet Européen publié sous le numéro 0146455. Ce dérivé fonctionnalisé du dextrane inhibe la prolifération des cellules musculaires lisses, comme le montre la figure 2, mais inhibe aussi l'activation du complément (tests de CH50 réalisés) et stimule la prolifération des cellules endothéliales.

### 2) Protocole

Une solution de DMCBSu, tel que celui décrit ci-dessus, à 16% (m/V) est préparée dans un tampon MES 0.01 M, pH 3.5. L'agent de couplage EDAC est ajouté à 13% (m/V) à la solution et laissé 5 minutes sous agitation. Le second agent de couplage NHS est ajouté à 6% (m/V) et laissé 30 minutes sous agitation.

Les pions d'aciers sont introduits dans 1 ml de tampon phosphate 0,1 M, pH 7,2 auquel est ajoutée la solution décrite ci-dessus. Le pH est ramené à 8.

La réaction se poursuit pendant 24 heures puis les pions sont rincés à l'eau bidistillée. Les pions sont alors conservés à 50°C dans l'étuve à vide.

### EXEMPLE 7 : Greffage d'un dérivé fonctionnalisé de dextrane sur une surface métallique aminée.

Le support, les agents de couplage et le protocole sont identiques à ceux décrits dans l'exemple 6.

A la différence de l'exemple 6, le dérivé fonctionnalisé de dextrane utilisé est le DMC_{0,68}B_{0,34}Su_{0,12} ; il répond à la formule générale DMCₐB_{b}Su_{c}S_{d} telle que celle précédemment décrite.

Son protocole de préparation est tel que décrit dans le brevet Européen publié sous le numéro 0146455. Ce dérivé fonctionnalisé de dextrane inhibe la prolifération des cellules endothéliales de la lignée EAhy 926.

### EXEMPLE 8 : Greffage d'un dérivé fonctionnalisé de dextrane sur une surface métallique aminée.

Le support, les agents de couplage et le protocole sont identiques à ceux décrits dans l'exemple 6.

A la différence de l'exemple 6, le dérivé fonctionnalisé de dextrane utilisé est le DMC_{0,6}B_{0,45}Su_{0,65} de masse molaire moyenne en poids de 100 000 g/mol ; il répond à la formule générale DMCₐB_{b}Su_{c}S_{d} telle que celle précédemment décrite.

Son protocole de préparation est tel que décrit dans le brevet Européen publié sous le numéro 0146455.

Son activité spécifique anticoagulante est de 4,3 Ul/mg par comparaison à l'héparine dont l'activité est de 173 Ul/mg. Ce composé est stérilisé par filtration et lyophilisation ; les tests de stérilité et d'apyrogènicité sont conformes.

### EXEMPLE 9 : Greffage d'un dérivé fonctionnalisé de dextrane sur une surface métallique aminée.

Le support, les agents de couplage et le protocole sont identiques à ceux décrits dans l'exemple 6.

A la différence de l'exemple 6, le dérivé fonctionnalisé de dextrane utilisé est le DMC_{0,61}B_{0,39}Su_{0,23} ; il répond à la formule générale DMCₐB_{b}Su_{c}S_{d} telle que celle précédemment décrite.

Son protocole de préparation est tel que décrit dans le brevet Européen publié sous le numéro 0146455.

Son activité spécifique anticoagulante est de 3,5 Ul/mg par comparaison à l'héparine dont l'activité est de 173 Ul/mg. Ce composé est stérilisé par filtration et lyophilisation ; les tests de stérilité et d'apyrogènicité sont conformes.

### EXEMPLE 10 : Test d'adhérence et de croissance des cellules endothéliales sur les pions d'acier inox 316 L modifiés.

### 1) Test, cellules, témoins et échantillons utilisés

Ce test est une mesure directe de l'adhérence et de la prolifération des cellules endothéliales de la lignée EAhy 926, déjà décrites précédemment. Ce test est conforme à la norme internationale ISO 10 993-5, concernant l'évaluation biologique des dispositifs médicaux.

Les supports utilisés sont des pions d'acier inox 316L sur lesquels out été effectuées des modifications de surface.

Les supports témoins sont des puits des plaques de culture cellulaires 24 puits commercialisées par Corning Costar.

Le milieu de culture utilisé est du milieu de culture à 10% (V/V) en sérum de veau foetal (SVF).

Les pions utilisés sont des pions bruts, aminés et recouverts du dérivé fonctionnalisé de dextrane (DMC0,8B_{0,22}Su_{0,11}) précédemment décrit.

### 2) Protocole

Le DMCBSu est greffé sur des pions d'acier inox 316 L.

8 pions sont préparés afin d'effectuer des mesures d'adhérence des cellules et d'évaluer leur croissance en fonction du support. 8 pions témoins sont ajoutés au test ainsi que 8 puits vierges de plaques 24 puits stériles de chez Corning Costar.

Les pions greffés et les pions bruts sont stérilisés en fond de puits 40 mn dans de l'Ethanol 70°. Ils sont ensuite rincés 2 fois au PBS stérile puis passivés avec du milieu de culture (10% de SVF) durant 3 jours.

Les cellules sont ensemencées à 25 000 cellules par puits par pions.

Deux pions sont prélevés et analysés à 24 h, 48 h, 72 h et 7 jours.

L'analyse est effectuée comme précédemment, par décollement des cellules adhérentes sur les pions et comptage au coulter.

### 3) Résultats

Les résultats du test sont donnés dans le tableau 2. Les résultats ont été ramenés en nombre de cellules par cm². La surface d'une face de pion d'acier inox 316L mesure le quart de celle d'un fond de puits (2 cm²). Il a été considéré que les cellules adhéraient, au départ, deux fois plus sur le pion que sur le puits car elles ont été déposées sur le pion puis immergées dans le milieu.

Une meilleure adhérence des cellules a lieu sur les pions greffés que sur les pions bruts.

Ces résultats indiquent une efficacité du greffage et une action du DMC_{0,8}B_{0,22}Su_{0,11} bien qu'il soit immobilisé sur une surface.

On obtient des résultats identiques avec le composé DMC_{0,6}B_{0,45}Su_{0,65} précédemment décrit.

**Tableau 2 :**

| *Nombre de cellules par cm*^{*2*} *en fonction du temps et de la surface* | | | |
|---|---|---|---|
| | Pions greffés | Pions témoins | Puits témoins |
| 24 heures | 17127 | 9500 | 3878 |
| 48 heures | 20047 | 13470 | 6233 |
| 72 heures | 28840 | 15540 | 11072 |
| Tt0 | 25000 | 25000 | 12500 |

### EXEMPLE 11 : Exemple de test d'adhérence des cellules endothéliales sur les pions d'acier inox 316 L modifiés

Les principaux agents de ce test biologique sont identiques à ceux de l'exemple 10. A la différence de l'exemple 10, le dérivé fonctionnalisé du dextrane est le DMC_{0,68}B_{0,34}Su_{0,12}.

Les cellules ont été ensemencées à une densité de 75 000 cellules par puits par pion. Une seule mesure a été effectuée au bout de 5 jours d'incubation.

Les résultats sont donnés dans le tableau 3.

**Tableau 3 :**

| *Nombre de cellules par cm*^{*2*} *après 5 jours d'incubation sur les pions* | |
|---|---|
| | (Cellules)/cm² |
| Puits témoins | 148160 |
| Pion oxydé | 122000 |
| Pion aminé | 118500 |
| Pion brut | 120040 |
| Pion greffé | 65760 |
| Puits des pions bruts | 127410 |
| Puits des pions greffés | 139860 |

Les différents pions intermédiaires n'influent que peu sur l'adhérence des cellules endothéliales. Cependant, le dérivé fonctionnalisé de dextrane greffé à la surface du pion inhibe l'adhérence des cellules EAhy 926.

Ces résultats confirment l'efficacité du greffage par la méthode décrite dans ce document.

De plus, les dérivés fonctionnalisés du dextrane ont des effets sur l'activation du complément et sur l'activation plaquettaire qui sont l'objet de mesures.

### EXEMPLE 12 : Exemple de test d'adhérence des cellules endothéliales et de cellules musculaires lisses sur les pions d'acier inox 316 L modifiés

Les principaux agents de ce test biologique sont identiques à ceux de l'exemple 10. A la différence de l'exemple 10, le dérivé fonctionnalisé du dextrane est le DMC_{0,6}B_{0,45}Su_{0,65}.

Les cellules ont été ensemencées à une densité de 10 000 cellules endothéliales par puits par pion et 15 000 cellules musculaires lisses par puits par pion.

Une seule mesure a été effectuée au bout de 5 jours d'incubation.

Les résultats sont donnés dans le tableau 4.

**Tableau 4 :**

| *Nombre de cellules après 5 jours d'incubation sur les pions* | | |
|---|---|---|
| | Cellules endothéliales | CML |
| Pion brut | 5 000 | 315 000 |
| Pion oxydé | 4 800 | 300 000 |
| Pion aminé | 48 800 | 160 000 |
| Pion greffé | 51 000 | 87 500 |

Une stimulation de la croissance des cellules endothéliales et une inhibition de la croissance des cellules musculaires lisses sont observées. Ces résultats sont obtenus à la fois sur les pions aminés et sur les pions greffés, le revêtement aminé a donc également un effet en tant que tel.

### EXEMPLE 13 : Préparation d'endoprothèses vasculaires selon la présente invention, nettoyage, oxydation, amination de la surface et greffage de dérivés fonctionnalisés du dextrane.

Le support utilisé consiste en des stents, expansibles par ballonnet, d'acier inoxydable 316L fournis par la société Sofradim. L'acier inox 316L est un acier austénitique type 18/12 (teneur en Chrome/Nickel), à structure CFC répondant aux normes européennes NF S 90 401, NF S 90 402, NF S 90 403 et NF S 94 051.

Les solutions de nettoyage sont des solutions d'acétone et d'éthanol chaud.

La solution oxydante est un mélange sulfochromique.

Le dérivé silanique, est le γ-APS (3-aminopropyltriéthoxysilane). Ce composé se fixe sur les fonctions hydroxyles de la surface et permet d'obtenir les fonctions amines apparentes.

Les dérivés fonctionnalisés du dextrane utilisés sont : DMC_{0,8}B_{0,22}Su_{0,11}, DMC_{0,6}B_{0,45}Su_{0,65}, DMC_{0,68}B_{0,34}Su_{0,12}, DMC_{0,61}B_{0,39}Su_{0,23}, décrits précédemment.

### 2) Protocole.

Les stents d'acier inox 316L sont soumis à la sonication dans l'acétone 10 mn puis placés 10 mn dans une solution d'éthanol à 70°C. Cette étape permet l'élimination des traces d'éléments tensioactifs qui pourraient être restés à la surface suite au nettoyage.

Une solution de γ-APS à 10% (V/V) dans l'éthanol 95° est préparée et laissée 1 heure sous agitation afin d'hydrolyser les fonctions éthoxy et de permettre un greffage sur des fonctions hydroxyles.

La solution d'oxydation est une solution sulfochromique à 2,7% (m/V) en bichromate de potassium dans l'acide sulfurique 80%.

Dans le même temps, les stents d'acier nettoyés sont placés dans la solution d'oxydation et laissés 1 heure sous faible agitation.Les stents oxydés sont ensuite rincés dans l'eau bidistillée, 5 minutes sous sonication. Les stents ainsi récupérés sont alors placés dans la solution de dérivés silanique et laissés 1 heure sous faible agitation.

On effectue ensuite un recuit des stents soumis au γ-APS pour permettre le retournement de la molécule de γ-APS selon la réaction illustrée précédemment. La phase aqueuse est soigneusement retirée à l'aide d'une pipette sans entrer en contact avec les stents d'acier puis le récipient contenant les stents est placé 10 minutes à 120°C. Les stents sont alors rincés à l'eau bidistillée.

Des solutions de DMCBSu, tels que ceux décrits ci-dessus, à 16% (mN) sont préparées dans un tampon MES 0.01 M, pH 3.5. L'agent de couplage EDAC est ajouté à 13% (m/V) à la solution et laissé 5 minutes sous agitation. Le second agent de couplage NHS est ajouté à 6% (m/V) et laissé 30 minutes sous agitation.

Les stents sont introduits dans 1 mL de tampon phosphate 0,1M, pH 7,2 auquel est ajoutée la solution décrite ci-dessus.

Le pH est ramené à 8

La réaction est laissée 24 heures puis les stents sont rincés à l'eau bidistillée. Les stents sont alors conservés à 50°C dans l'étuve à vide.

Cette étape de greffage peut être effectuée plusieurs fois pour améliorer l'épaisseur de la couche active.

Des analyses XPS des surfaces et des observations au Microscope Electronique à Balayage (MEB) ont montré, sur toute les surfaces, un recouvrement homogène de γ-APS d'épaisseur supérieure à 10 nm ainsi que la présence homogène des groupements chimiques caractéristiques des dérivés fonctionnalisés du dextrane utilisés.

## Revendications

1. Procédé de revêtement et liaison de la surface d'un substrat métallique avec une couche d'un composé polysaccharidique, **caractérisé en ce que**, à partir du substrat métallique :
(a) on dispose d'un agent de modification chimique de la surface du substrat métallique, par exemple sous forme liquide ;
(b) on dispose d'un agent de recouvrement intermédiaire, comprenant un composé silanique, en solution par exemple, comportant deux restes réactifs, l'un avec le substrat métallique, et l'autre, directement ou indirectement, avec le composé polysaccharidique, ledit composé silanique comportant un ou plusieurs groupes aminés ou dérivés d'amines et un ou plusieurs groupes hydroxyles ou alcoxy ;
(c) on dispose d'un agent de revêtement, comprenant, en solution par exemple, le composé polysaccharidique ;
et on effectue les opérations suivantes :
(1) on met en contact la surface du substrat métallique, avec l'agent de modification chimique, pour obtenir une surface modifiée chimiquement ;
(2) on met en contact la surface modifiée chimiquement avec l'agent de recouvrement intermédiaire, pour obtenir une surface revêtue avec une couche intermédiaire comprenant le composé silanique, lié par covalence au substrat métallique ;
(3) le substrat métallique dont la surface est revêtue avec la couche intermédiaire est recuit, avant mise en contact avec l'agent de revêtement ;
(4) on met en contact la couche intermédiaire avec l'agent de revêtement, pour revêtir ladite couche intermédiaire avec un revêtement comprenant le composé polysaccharidique lié par covalence, directement ou indirectement, au composé silanique.

2. Procédé selon la revendication 1, **caractérisé en ce que** la surface modifiée chimiquement est nettoyée avant mise en contact avec l'agent de recouvrement intermédiaire, par exemple avec une solution d'acétone, ou d'alcool, et/ou d'agents tensioactifs.

3. Procédé selon la revendication 1, **caractérisé en ce que** le recuit est effectué à une température comprise entre 80 et 140°C, et/ou pendant une durée comprise entre 1 et 30 minutes.

4. Procédé selon la revendication 1, **caractérisé en ce que** la surface revêtue de la couche intermédiaire est lavée, avant mise en contact avec l'agent de revêtement.

5. Procédé selon la revendication 1, **caractérisé en ce que** la surface revêtue avec le composé polysaccharidique est lavée.

6. Procédé selon la revendication 1, **caractérisé en ce que** l'opération (1) et/ou (2) est mise en oeuvre en phase liquide ou vapeur.

7. Procédé selon la revendication 1, **caractérisé en ce que** l'opération (2) est effectuée en phase liquide, à un pH compris entre 2 et 9, et/ou à une température comprise entre 25 et 120°C, et/ou pendant une durée comprise entre 1 et 120 minutes.

8. Procédé selon la revendication 1, **caractérisé en ce que** l'opération (2) est effectuée en phase vapeur, à une température supérieure à 120 °C et à une pression supérieur à 4 mbar.

9. Procédé selon la revendication 1, **caractérisé en ce que** l'agent de modification chimique, sous forme liquide, comprend au moins un acide minéral fort, par exemple acide sulfurique, ou chlorhydrique, ou nitrique, et au moins un oxyde de chrome.

10. Procédé selon la revendication 9, **caractérisé en ce que** l'acide minéral fort est présent dans l'agent de modification chimique, sous forme liquide, dans la proportion comprise entre 5 et 100 % (VN)

11. Procédé selon la revendication 9, **caractérisé en ce que** le ou les oxydes de chrome sont présents dans l'agent de modification chimique, sous forme liquide, dans la proportion comprise entre 1 et 40 % (mN).

12. Procédé selon la revendication 9, **caractérisé en ce que** l'oxyde de chrome a une masse molaire moyenne comprise entre 50 et 500 g/mol, et est par exemple choisi dans le groupe constitué par les bichromates de potassium et les oxydes de chrome IV.

13. Procédé selon la revendication 9, **caractérisé en ce que** le pH de l'agent de modification chimique, sous forme liquide, est compris entre 1 et 6.

14. Procédé selon la revendication 1, **caractérisé en ce que** l'agent de recouvrement intermédiaire, à l'état liquide, comprend au plus 50%, et de préférence entre 1 et 30 % (V/V) du composé silanique.

15. Procédé selon la revendication 1, **caractérisé en ce que** le composé silanique a une masse molaire moyenne comprise entre 50 et 500 g/mol.

16. Procédé selon la revendication 1, **caractérisé en ce que** le composé silanique est choisi dans la groupe constitué par les aminopropylsilanes, et les aminobutylsilanes.

17. Procédé selon la revendication 1, **caractérisé en ce que** que l'agent du revêtement, à l'état liquide, comprend entre 1 et 20 % (m/V) du composé polysaccharidique, en solution.

18. Procédé selon la revendication 1, **caractérisé en ce que** l'agent de revêtement comprend un polysaccharide non modifié ou non fonctionnalisé, par exemple un dextrane, et/ou un dérivé polysaccharidique fonctionnalisé, susceptible d'être obtenu à partir d'un polysaccharide, par exemple un dextrane.

19. Procédé selon la revendication 18, **caractérisé en ce que** le polysaccharide est un dextrane ayant une masse molaire moyenne comprise entre 20.000 et 1.000.000 g/mol, par exemple ayant une masse molaire égale à 40.000 ou 70.000, ou 460.000 g/mol.

20. Procédé selon la revendication 1, **caractérisé en ce que** l'agent de revêtement comprend au moins un agent de couplage, par exemple choisi dans le groupe constitué par le bis-sulfo (succinimide-subérate) en abrégé BS3, le diméthyladipimidate en abrégé DMA, l'époxirane, le bis-époxirane, les succinimides, l'épichlorydrine, les carbodiimides.

21. Procédé selon la revendication 20, **caractérisé en ce que** l'agent de couplage est le 1-éthyl-3-3-(diméthylaminipropyl)-carbodiimide, en abrégé EDAC, ou le N-hydroxysuccinimide, en abrégé NHS.

22. Procédé selon la revendication 21, **caractérisé en ce que** l'agent de couplage est présent, dans l'agent de revêtement, à proportion de 20 à 50 moles pour 100 moles du motif osidique de la chaîne polysaccharidique.

23. Procédé selon la revendication 18, **caractérisé en ce que** l'agent de revêtement comprend un polysaccharide supplémentaire, naturel ou synthétique, substitué par des fonctions carboxylates et/ou sulfates, ledit polysaccharide supplémentaire étant différent dudit dérivé polysaccharidique fonctionnalisé.

24. Substrat métallique revêtu, susceptible d'être obtenu par un procédé selon l'une quelconque des revendications 1 à 23.

25. Endoprothèse vasculaire, du type stent, comprenant un substrat métallique revêtu selon la revendication 24.

26. Endoprothèse vasculaire selon la revendication 25, **caractérisée en ce que** le substrat métallique est un alliage, par exemple un acier inoxydable, ou un superalliage, par exemple le phynox.

## Patentansprüche

1. Verfahren zum Beschichten und Verbinden der Oberfläche eines metallischen Substrats mit einer Schicht einer Polysaccharidverbindung, **dadurch gekennzeichnet, dass**, ausgehend von dem Metallsubstrat:
(a) ein Mittel zur chemischen Modifikation der Oberfläche des metallischen Substrats, zum Beispiel in flüssiger Form, bereitgestellt wird;
(b) ein Mittel zum Zwischenbeschichten bereitgestellt wird, das eine Silanverbindung, zum Beispiel in Lösung, aufweist, die zwei reaktive Reste aufweist, von denen einer mit dem metallischen Substrat und der andere direkt oder indirekt mit der Polysaccharidverbindung reagieren kann, wobei die Silanverbindung eine oder mehrere Amingruppen oder Derivate von Aminen und eine oder mehrere Hydroxyl- oder Alcoxygruppen aufweist;
(c) ein Mittel zum Beschichten bereitgestellt wird, das die Polysaccharidverbindung, zum Beispiel in Lösung, aufweist;
und dass die folgenden Schritte ausgeführt werden:
(1) die Oberfläche des metallischen Substrats wird mit dem Mittel zur chemischen Modifikation in Berührung gebracht, um eine chemisch modifizierte Oberfläche zu erhalten;
(2) die chemisch modifizierte Oberfläche wird mit dem Mittel zum Zwischenbeschichten in Berührung gebracht, um eine mit einer Zwischenschicht beschichtete Oberfläche zu erhalten, die die Silanverbindung aufweist, die kovalent an das metallischen Substrat gebunden ist;
(3) das metallische Substrat, dessen Oberfläche mit einer Zwischenschicht beschichtet ist, wird, vor dem In-Berührung-Bringen mit dem Mittel zum Beschichten geglüht;
(4) die Zwischenschicht wird mit dem Mittel zum Beschichten in Berührung gebracht, um die Zwischenschicht mit einer Beschichtung zu beschichten, die die Polysaccharidverbindung aufweist, die direkt oder indirekt kovalent an die Silanverbindung gebunden ist.

2. Verfahren nach Anspruch 1, **dadurch gekennzeichnet, dass** die chemisch modifizierte Oberfläche vor dem In-Berührung-Bringen mit dem Mittel zum Zwischenbeschichten, zum Beispiel mit einer Aceton- oder Alkohollösung und/oder einer Lösung von grenzflächenaktiven Mitteln gereinigt wird.

3. Verfahren nach Anspruch 1, **dadurch gekennzeichnet, dass** das Glühen bei einer Temperatur von 80 bis 140°C und/oder für einen Zeitraum von 1 bis 30 Minuten durchgeführt wird.

4. Verfahren nach Anspruch 1, **dadurch gekennzeichnet, dass** die mit der Zwischenschicht beschichtete Oberfläche vor dem In-Berührung-Bringen mit dem Beschichtungsmittel gewaschen wird.

5. Verfahren nach Anspruch 1, **dadurch gekennzeichnet, dass** die mit der Polysaccharidverbindung beschichtete Oberfläche gewaschen wird.

6. Verfahren nach Anspruch 1, **dadurch gekennzeichnet, dass** der Schritt (1) und/oder (2) in flüssiger Phase oder in der Dampfphase durchgeführt wird.

7. Verfahren nach Anspruch 1, **dadurch gekennzeichnet, dass** der Schritt (2) in flüssiger Phase bei einem pH-Wert von 2 bis 9 und/oder einer Temperatur von 25 bis 120°C und/oder für einen Zeitraum von 1 bis 120 Minuten durchgeführt wird.

8. Verfahren nach Anspruch 1, **dadurch gekennzeichnet, dass** der Schritt (2) in der Dampfphase, bei einer Temperatur über 120°C und bei einem Druck von mehr als 4 mbar durchgeführt wird.

9. Verfahren nach Anspruch 1, **dadurch gekennzeichnet, dass** das Mittel zur chemischen Modifikation in flüssiger Form zumindest eine starke Mineralsäure, zum Beispiel Schwefelsäure oder Salzsäure oder Salpetersäure, und zumindest ein Chromoxid aufweist.

10. Verfahren nach Anspruch 9, **dadurch gekennzeichnet, dass** die starke Mineralsäure in dem Mittel zur chemischen Modifikation in flüssiger Form in einem Anteil von 5 bis 100 % (V/V) vorliegt.

11. Verfahren nach Anspruch 9, **dadurch gekennzeichnet, dass** das oder die Chromoxide in dem Mittel zur chemischen Modifikation in flüssiger Form in einem Anteil von 1 bis 40 % (m/V) vorliegen.

12. Verfahren nach Anspruch 9, **dadurch gekennzeichnet, dass** das Chromoxid eine mittlere Molmasse zwischen 50 und 500 g/mol aufweist und zum Beispiel ausgewählt ist aus der Gruppe bestehend aus den Kaliumbichromaten und den Oxiden von Chrom IV.

13. Verfahren nach Anspruch 9, **dadurch gekennzeichnet, dass** der pH-Wert des Mittels zur chemischen Modifikation in flüssiger Form bei 1 bis 6 liegt.

14. Verfahren nach Anspruch 1, **dadurch gekennzeichnet, dass** das Mittel zum Zwischenbeschichten in flüssigem Zustand höchstens 50 % und vorzugsweise 1 bis 30 % (V/V) der Silanverbindung aufweist.

15. Verfahren nach Anspruch 1, **dadurch gekennzeichnet, dass** die Silanverbindung eine mittlere Molmasse von 50 bis 500 g/mol aufweist.

16. Verfahren nach Anspruch 1, **dadurch gekennzeichnet, dass** die Silanverbindung ausgewählt ist aus der Gruppe bestehend aus den Aminopropylsilanen und den Aminobutylsilanen.

17. Verfahren nach Anspruch 1, **dadurch gekennzeichnet, dass** das Mittel zum Beschichten in flüssigem Zustand 1 bis 20 % (m/V) der Polysaccharidverbindung in Lösung aufweist.

18. Verfahren nach Anspruch 1, **dadurch gekennzeichnet, dass** das Mittel zum Beschichten ein nicht modifiziertes oder nicht funktionalisiertes Polysaccharid, zum Beispiel ein Dextran und/oder ein funktionalisiertes Polysaccharidderivat aufweist, das ausgehend von einem Polysaccharid, zum Beispiel einem Dextran, erhalten werden kann.

19. Verfahren nach Anspruch 18, **dadurch gekennzeichnet, dass** das Polysaccharid ein Dextran ist, das eine mittlere Molmasse von 20.000 bis 1.000.000 g/mol, zum Beispiel eine Molmasse von 40.000 oder 70.000 oder 460.000 g/mol aufweist.

20. Verfahren nach Anspruch 1, **dadurch gekennzeichnet, dass** das Mittel zum Beschichten zumindest ein Kopplungsmittel aufweist, das zum Beispiel ausgewählt ist aus der Gruppe bestehend aus Bis(sulfosuccinimidyl)suberat, abgekürzt BS3, Dimethyladipimidat, abgekürzt DMA, Epoxiran, Bisepoxiran, den Succinimiden, Epichlorydrin und den Carbodiimiden.

21. Verfahren nach Anspruch 20, **dadurch gekennzeichnet, dass** das Kopplungsmittel 1-Ethyl-3-3-(dimethylaminopropyl)-carbodiimid, abgekürzt EDAC, oder N-Hydroxysuccinimid, abgekürzt NHS, ist.

22. Verfahren nach Anspruch 21, **dadurch gekennzeichnet, dass** das Kopplungsmittel in dem Mittel zum Beschichten in einem Anteil von 20 bis 50 mol pro 100 mol des Zuckermotives der Polysaccharidkette vorliegt.

23. Verfahren nach Anspruch 18, **dadurch gekennzeichnet, dass** das Mittel zum Beschichten ein zusätzliches natürliches oder synthetisches Polysaccharid aufweist, das mit Carboxylat- oder Sulfatfunktionen substituiert ist, wobei sich das zusätzliche Polysaccharid von dem funktionalisierten Polysaccharidderivat unterscheidet.

24. Beschichtetes metallisches Substrat, das durch ein Verfahren nach einem der Ansprüche 1 bis 23 erhältlich ist.

25. Vaskuläre Endoprothese vom Typ Stent, die ein beschichtetes metallisches Substrat nach Anspruch 24 aufweist.

26. Vaskuläre Endoprothese nach Anspruch 25, **dadurch gekennzeichnet, dass** das metallische Substrat eine Legierung, zum Beispiel ein rostfreier Stahl, oder eine Superlegierung, zum Beispiel Phynox, ist.

## Claims

1. A method of coating and bonding the surface of a metallic substrate with a layer of a polysaccharide compound, **characterized in that**, on the basis of the metallic substrate:
(a) we have an agent for chemical modification of the surface of the metallic substrate, for example in liquid form;
(b) we have an agent for intermediate covering, comprising a silane compound, in solution for example, containing two reactive residues, one with the metallic substrate, and the other, directly or indirectly, with the polysaccharide compound,said silane compound containing one or more amine groups or derivatives of amines and one or more hydroxyl or alkoxy groups;
(c) we have a coating agent, containing, in solution for example, the polysaccharide compound;
and the following operations are carried out:
(1) the surface of the metallic substrate is brought into contact with the agent for chemical modification, to obtain a chemically modified surface;
(2) the chemically modified surface is brought into contact with the intermediate covering agent, to obtain a surface coated with an intermediate layer comprising the silane compound, bound covalently to the metallic substrate;
(3) the metallic substrate whose surface is coated with the intermediate layer is annealed, before being brought into contact with the coating agent;
(4) the intermediate layer is brought into contact with the coating agent, to coat said intermediate layer with a coating comprising the polysaccharide compound bound covalently, directly or indirectly, to the silane compound.

2. The method as claimed in claim 1, **characterized in that** the chemically modified surface is cleaned before being brought into contact with the intermediate covering agent, for example with a solution of acetone, or of alcohol, and/or of surfactants.

3. The method as claimed in claim 1, **characterized in that** annealing is carried out at a temperature between 80 and 140°C and/or for a time between 1 and 30 minutes.

4. The method as claimed in claim 1, **characterized in that** the surface coated with the intermediate layer is washed, before being brought into contact with the coating agent.

5. The method as claimed in claim 1, **characterized in that** the surface coated with the polysaccharide compound is washed.

6. The method as claimed in claim 1, **characterized in that** operation (1) and/or (2) is carried out in the liquid or vapor phase.

7. The method as claimed in claim 1, **characterized in that** operation (2) is carried out in the liquid phase, at a pH between 2 and 9, and/or at a temperature between 25 and 120°C, and/or for a time between 1 and 120 minutes.

8. The method as claimed in claim 1, **characterized in that** operation (2) is carried out in the vapor phase, at a temperature above 120°C and at a pressure greater than 4 mbar.

9. The method as claimed in claim 1, **characterized in that** the agent for chemical modification, in the liquid form, contains at least one strong inorganic acid, for example sulfuric, hydrochloric or nitric acid, and at least one oxide of chromium.

10. The method as claimed in claim 9, **characterized in that** the strong inorganic acid is present in the agent for chemical modification, in the liquid form, in a proportion between 5 and 100% (v/v).

11. The method as claimed in claim 9, **characterized in that** the oxide or oxides of chromium are present in the agent for chemical modification, in the liquid form, in a proportion between 1 and 40% (w/v).

12. The method as claimed in claim 9, **characterized in that** the oxide of chromium has an average molecular weight between 50 and 500 g/mol, and is for example selected from the group consisting of the potassium dichromates and the chromium (IV) oxides.

13. The method as claimed in claim 9, **characterized in that** the pH of the agent for chemical modification, in the liquid form, is between 1 and 6.

14. The method as claimed in claim 1, **characterized in that** the intermediate covering agent, in the liquid state, contains at most 50%, and preferably between 1 and 30% (v/v) of the silane compound.

15. The method as claimed in claim 1, **characterized in that** the silane compound has an average molecular weight between 50 and 500g/mol.

16. The method as claimed in claim 1, **characterized in that** the silane compound is selected from the group consisting of the aminopropysilanes and the aminobutysilanes.

17. The method as claimed in claim 1, **characterized in that** the coating agent, in the liquid state, contains between 1 and 20% (w/v) of the polysaccharide compound, in solution.

18. The method as claimed in claim 1, **characterized in that** the coating agent contains an unmodified or unfunctionalized polysaccharide, for example a dextran, and/or a functionalized polysaccharide derivative that can be obtained from a polysaccharide, for example a destran.

19. The method as claimed in claim 18, **characterized in that** the polysaccharide is a dextran having an average molecular weight between 20 000 and 1 000 000 g/mol, for example having a molecular weight equal to 40 000 or 70 000, or 460 000 g/mol.

20. The method as claimed in claim 1, **characterized in that** the coating gent contains at least one coupling agent, for example selected from the group comprising bis-sulfo (succinimide-suberate) abbreviated to BS3, dimethyladipimidate abbreviated to DMA, epoxirane, bis-epoxirane, succinimides, epichlorohydrin, carbodiimides.

21. The method as claimed in claim 20, **characterized in that** the coupling agent is 1-ethyl-3-3- (dimethylaminopropyl)-carboddimide, abbreviated to EDAC, or N-hydrosuccinimide, abbreviated to NHS.

22. The method as claimed in claim 21, **characterized in that** the coupling agent is present, in the coating agent, in a proportion from 20 to 50 mol per 100 mol of the oside unit of the polysaccharide chain.

23. The method as claimed in claim 18, **characterized in that** the coating agent contains an additional polysaccharide, natural or synthetic, substitiued by carboxylate and/or sulfate functions, said additional polysaccharide being different from said functionalized polysaccharide derivative.

24. A coated metallic substrate, obtainable by a method as claimed in any one of the claims 1 to 23.

25. An endovascular prosthesis, of the stent type, comprising a coated metallic substrate as claimed in claim 24.

26. The endovascular prosthesis as claimed in claim 25, **characterized in that** the metallic substrate is an alloy, for example a stainless steel, or a superalloy, for example Phynox.
